(19) Europäisches Patentamt
European Patent Office
Office européen des brevets

(11) Publication number: 0 515 927 A2

# (12) EUROPEAN PATENT APPLICATION

(21) Application number: 92108232.7

(22) Date of filing: 15.05.92

(51) Int. Cl.[5]: C07H 15/244, A61K 31/70, C12P 19/56, C12N 1/20, //(C12P19/56,C12R1:03), (C12N1/20,C12R1:03)

(30) Priority: 29.05.91 US 706668
22.07.91 US 733556

(43) Date of publication of application: 02.12.92 Bulletin 92/49

(84) Designated Contracting States: AT BE CH DE DK ES FR GB GR IT LI LU MC NL PT SE

(71) Applicant: Bristol-Myers Squibb Company
345 Park Avenue
New York, N.Y. 10154(US)

(72) Inventor: Tenmyo, Osamu
28-91, Tsutsujigaoka
Midori-ku, Yokohama(JP)
Inventor: Saitoh, Kyoichiro
2-8-19, Numama
Zushi(JP)
Inventor: Hatori, Masami
2-16-12, Kurihamadai
Yokosuka(JP)
Inventor: Furumai, Tamotsu
436 Iwai-cho, Hodogaya-ku
Yokohama(JP)
Inventor: Oki, Toshikazu
4-20-10, Shodo
Sakae-ku, Yokohama(JP)

(74) Representative: Kinzebach, Werner, Dr. et al
Patentanwälte Reitstötter, Kinzebach und Partner Sternwartstrasse 4 Postfach 86 06 49
W-8000 München 86(DE)

(54) **Pradimicin antibiotics.**

(57) Pradimicin S of the formula

EP 0 515 927 A2

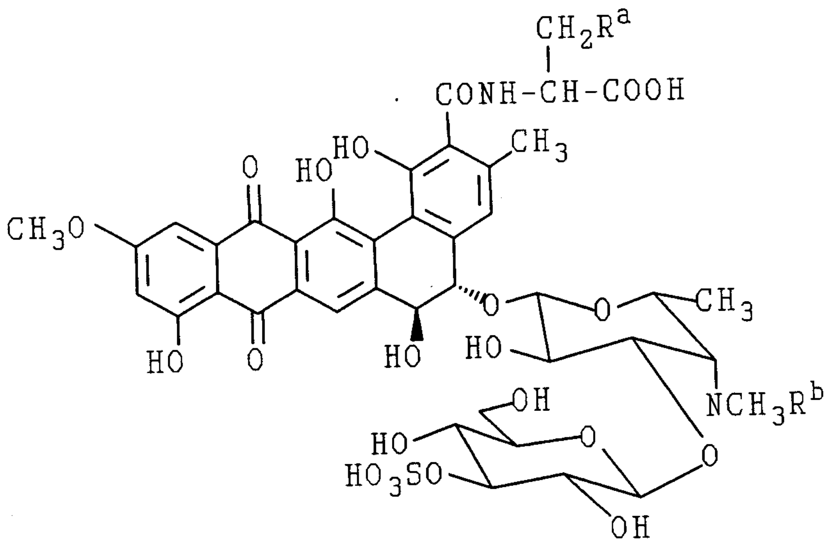

in which $R^a$ is hydrogen or hydroxy and $R^b$ is hydrogen, having antifungal and antiviral activity is prepared by cultivation of Actinomadura sp. AA0851 in a nutrient medium containing assimilable sources of carbon and nitrogen.

This application is a continuation-in-part of U.S. Patent Application Ser. No. 706,668 filed on May 29, 1991 which is hereby incorporated by reference in its entirety.

The present invention provides pradimicins S and FS and their N-methyl derivatives of general formula II,

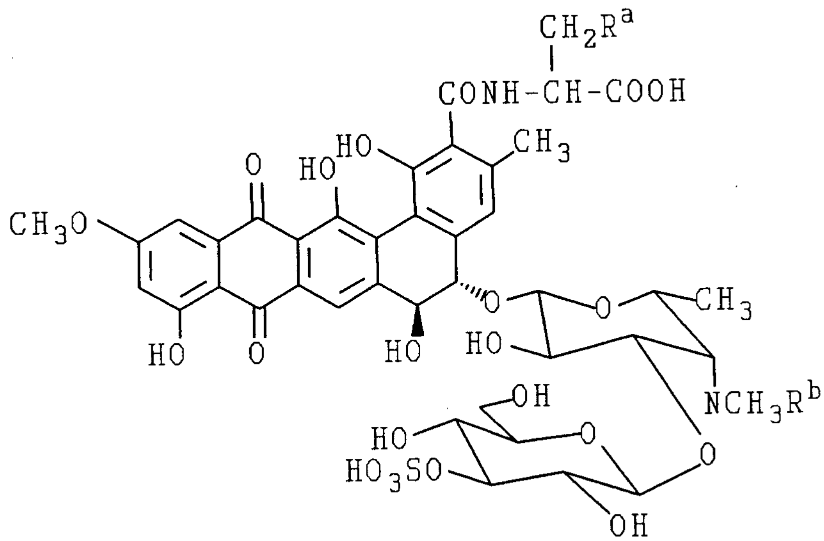

II

| | | |
|---|---|---|
| pradimicin S | $R^a$ = H, | $R^b$ = H |
| pradimicin FS | $R^a$ = OH, | $R^b$ = H |
| BMY-29273 | $R^a$ = H, | $R^b$ = $CH_3$ |

in which $R^a$ is hydrogen or hydroxy, $R^b$ is hydrogen or methyl and the alanine or the serine residue has the D-configuration. This invention is also concerned with a pharmaceutically acceptable salt thereof.

A further aspect of the invention provides a biologically pure culture of pradimicins S and FS-producing Actinomadura sp. AA0851.

Yet another aspect of the invention provides a process for preparing pradimicins S and FS which comprises cultivating the antibiotics-producing strain of Actinomadura sp. AA0851 under submerged and aerobic conditions in a medium containing assimilable sources of carbon, nitrogen, and D-alanine or D-serine.

Yet another aspect of the present invention provides a method for treating fungal or viral infections which comprises administering to a mammal so afflicted an antifungal or antiviral effective amount of pradimicin S or FS or its N-methyl derivative.

Yet another aspect of the invention provides a pharmaceutical formulation comprising pradimicin S or FS or its N-methyl derivative and a pharmaceutically acceptable carrier.

Fig. 1 is the IR spectrum of pradimicin S.
Fig. 2 is the $^1$H-NMR spectrum of pradimicin S.
Fig. 3 is the $^{13}$C-NMR spectrum of pradimicin S.
Fig. 4 is the UV spectrum of pradimicin S.
Fig. 5 is the IR spectrum of pradimicin FS.
Fig. 6 is the $^1$H-NMR spectrum of pradimicin FS.
Fig. 7 is the $^{13}$C-NMR spectrum of pradimicin FS.
Fig. 8 is the UV spectrum of pradimicin FS.

The present invention provides pradimicins S and FS and their N-methyl derivatives of general formula II,

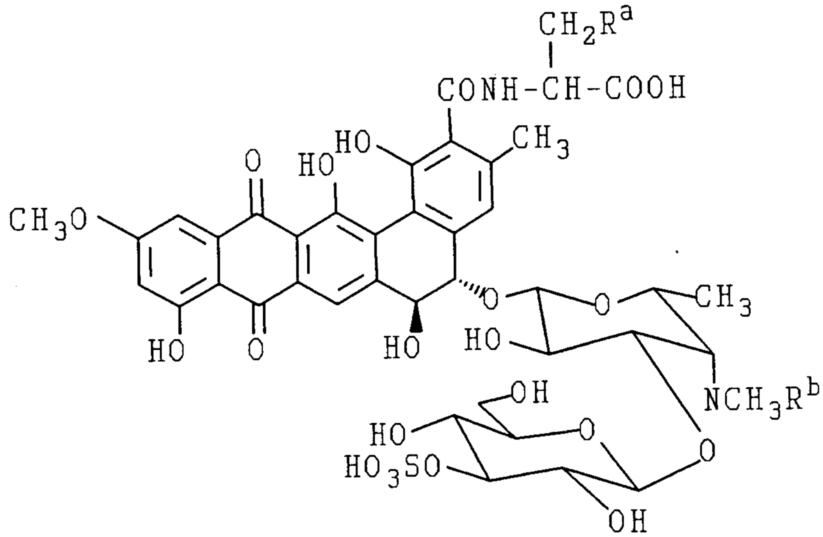

II

| | | |
|---|---|---|
| pradimicin S | $R^a$ = H, | $R^b$ = H |
| pradimicin FS | $R^a$ = OH, | $R^b$ = H |
| BMY-29273 | $R^a$ = H, | $R^b$ = $CH_3$ |

in which $R^a$ is hydrogen or hydroxy, $R^b$ is hydrogen or methyl and the alanine or the serine residue has the D-configuration.

A further aspect of the present invention provides a pharmaceutically acceptable salt thereof. More specifically, pradimicins S and FS and their N-methyl derivatives form pharmaceutically acceptable acid addition salts in which the anion does not contribute significantly to the toxicity of the salts and are compatible with the customary pharmaceutical vehicles and adapted for oral or parenteral administration. The pharmaceutically acceptable acid addition salts include the salts of pradimicin S or FS or N-methyl derivative thereof with mineral acids such as hydrochloric acid, hydrobromic acid, phosphoric acid and sulfuric acid, with organic carboxylic acids or organic sulfonic acids such as acetic acid, citric acid, maleic acid, succinic acid, benzoic acid, tartaric acid, fumaric acid, mandelic acid, ascorbic acid, malic acid, methanesulfonic acid, p-toluenesulfonic acid and other acids known and used in the galenic pharmacy. Preparation of these salts is carried out by conventional techniques involving reaction of pradimicin S or FS or N-methyl derivative thereof and the acid in a substantially equivalent amount.

Pradimicins S and FS and N-methyl derivatives thereof also form pharmaceutically acceptable metal and amine salts in which the cation does not contribute significantly to the toxicity or biological activity of the salts. These salts are also part of the present invention. Suitable metal salts include the sodium, potassium, calcium, barium, zinc and aluminum salts. The sodium or potassium salts are preferred. Amine salts are prepared from amines which are capable of forming stable salts with the acidic carboxy and sulfonic groups of pradimicin S or FS or N-methyl derivative thereof. Examples of such amines include, but are not limited to, triethylamine, procaine, dibenzylamine, N-benzyl-$\beta$-phenethylamine, 1-ephenamine, N,N'-dibenzylethylenediamine, dehydroabietylamine, N-ethylpiperidine, benzylamine and dicyclohexylamine.

Also included within the scope of the present invention are pharmaceutical formulations (compositions) comprising pradimicin S or FS or N-methyl derivative thereof or a pharmaceutically acceptable salt thereof. Preferably these formulations are in unit dosage forms such as tablets, pills, capsules, powders, granules, sterile parenteral solutions or suspensions, or suppositories for oral, parenteral or rectal administration. For preparing solid formulations such as tablets, the principal active ingredient is mixed with a pharmaceutical carrier, i.e., conventional tableting ingredients such as corn starch, lactose, sucrose, sorbitol, talc, stearic acid, magnesium stearate, dicalcium phosphate, gums, and other pharmaceutical diluents, e.g., water, to form a solid preformulation composition containing a homogeneous mixture of pradimicin S or FS or N-methyl derivative thereof or a pharmaceutically acceptable salt thereof. When referring to these preformulation compositions as homogeneous, it is meant that the active ingredient is dispersed evenly throughout the formulation so that the formulation may be readily subdivided into equally effective unit

dosage forms such as tablets, pills, capsules, and the like. This solid preformulation composition is then subdivided into unit dosage forms of the type described above containing from 0.1 to about 500 mg of the active ingredient of the present invention. The tablets or pills of the novel formulation can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures of polymeric acids with such materials as shellac, shellac and cetyl alcohol, cellulose acetate, and the like.

The liquid forms in which the novel formulation of the present invention may be incorporated for administration orally or by injection include aqueous solutions, suitably flavored syrups, aqueous or oil suspensions, flavored emulsions with edible oils such as cottonseed oil, sesame oil, coconut oil, peanut oil and the like, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinyl-pyrrolidone, gelatin and the like.

A further aspect of the invention provides a biologically pure culture of pradimicins S and FS-producing Actinomadura sp. AA0851.

Yet another aspect of the invention provides a process for preparing pradimicins S and FS which comprises cultivating the antibiotic-producing strain of Actinomadura sp. AA0851 under submerged and aerobic conditions in a medium containing assimilable sources of carbon and nitrogen.

## PRADIMICINS S and FS PRODUCING ORGANISM

## TAXONOMY

Strain AA0851 was isolated from a soil sample obtained at Manna Village, India on December, 1985.

Taxonomic studies on AA0851 were generally done by the methods adopted by the International Streptomyces Project (ISP) using the media recommended by Shirling-Gottlieb (Shirling, E. B. & D. Gottlieb: Methods for characterization of Streptomyces species. Intern. J. Syst. Bact. 16: 313-340, 1966), Waksman (Waksman, S. A.: In The Actinomycetes. Vol. II. Classification, identification and description of genera and species. pp. 328-334. The Williams & Wilkins Co., Baltimore, 1961) and Arai (Arai, T: In Culture Media for Actinomycetes. The Society for Actinomycetes Japan, 1975).

Characteristic whole-cell formulations were analyzed by using the procedures recommended by Becker and Lechevalier (Becker, B., M.P. Lechevalier, R.E. Gordon, and H.A. Lechevalier: Rapid differentiation between Nocardia and Streptomyces by paper chromatography of whole cell hydrolysates. Appl. Microbiol. 12: 421-423, 1964; Becker, B., M. P. Lechevalier, and H. A. Lechevalier: Chemical composition of cell-wall preparations from strains of various form-genera of aerobic actinomycetes. Appl. Microbiol. 13: 236-243, 1965). Phospholipid and mycolate composition were determined by the methods of Lechevalier et al (Lechevalier, M. P.: Identification of aerobic actinomycetes of clinical importance. J. Lab. Clin. Med. 71: 934-944, 1968; Lechevalier, M. P., C. Debievre and H. Lechevalier: Chemotaxonomy of aerobic actinomycetes: Phospholipid composition. Biochem. Syst. Ecol. 5: 249-260, 1977) and Minnikin et al (Minnikin, D. E., L. Alshamaony and M. Goodfellow: Differentiation of Mycrobacterium, Nocardia, and related taxa by thin-layer chromatographic analysis of whole-organism methanolysates. J. Gen. Microbiol. 88: 200-204, 1975), respectively. Menaquinone was analyzed by the procedures of Collins et al (Collins, M. D., H. N. Shah, and D. E. Minnikin: A note on the separation of natural mixtures of bacterial menaquinones using reverse-phase thin-layer chromatography. J. Appl. Bacteriol. 48: 277-282, 1980). The GC contents of the DNAs were isolated by the procedure of Furumai (Furumai, T., M. Sudoh, S. Sawairi and H. B. Maruyama: DNA homology studies in Streptomyces using S1 nuclease. J. Antibiotics 37: 641-645, 1984) and determined by the method of Tamaoka et al (Tamaoka, J. and K. Komagata: Determination of DNA base composition by reverse-phase high-performance liquid chromatography. FEMS microbiol. Lett. 25: 125-128, 1984).

The determination of color name and color cord was made by comparison of the cultures with color chips from the Manual of Color Names (Japan Color Enterprise Co., Ltd., 1987).

## Morphology

Strain AA0851 formed a branched vegetative mycelium with a sparse aerial mycelium. Fragmentation of vegetative mycelia did not appear either on an agar medium or in a liquid medium. Rudimentary and

retarded aerial mycelia were produced both on inorganic salts-starch agar (ISP-4) and on yeast starch agar. Straight chains of less than 20 spores were occasionally observed at the top of sporulating aerial mycelia on 1/2 inorganic salts-starch agar (ISP medium 4) supplemented with 0.1% yeast extract (Difco Laboratories, Detroit, Mich.), 0.00005% biotin, 0.00005% thiamine HCl, 0.00005% riboflavine, 0.00005% pyridoxine HCl, 0.00005% vitamin $B_{12}$ and 1.0% agar (designated 1/2 ISP-4). The spores were oval to elliptical in shape with spiny surface and measured to by 1.0-1.2 to 1.1-1.3 µm. Spores were not motile, and pseudosporangia were observed.

**Cell chemistry**

Upon hydrolysis, whole cells of strain AA0851 yielded meso-diaminopimelic acid, madurose, ribose, mannose, glucose and galactose. Consequently strain AA0851 has a type III cell wall with sugar pattern characteristic of type B.

Mycolic acids were not detected. By phospholipids analysis, the wall has a type P1 phospholipid pattern containing phosphatidylinositol mannoside, phosphatidylinositol and diphosphatidylglycerol. Analysis of the menaquinone composition revealed 50.9% MK-9 (H6), 30% MK-9 (H8), 11.4% MK-9 (H4), 4% MK-9 (H10), 3.1% MK-9 (H2) and 0.6% MK-9 (H12).

**Cultural characteristics**

Strain AA0851 showed better growth on organic agar media than media containing inorganic nitrogen sources at temperatures between 21°C and 45°C. Colonies grew slowly (1 to 2 weeks) and were convex with irregular margin. Mature aerial mycelia were generally powdery with white to light gray in color, but rarely white velvety to cottony on 1/2 ISP-4 agar medium supplemented with 0.1% yeast extract. These aerial mycelia sometimes became light gray after about 1 month at 28°C. The color of vegetative mycelia ranged from pale pink to dark red. Pale yellowish pink, grayish pink or dark red diffusible pigments were produced on various agar media. The color of both vegetative mycelia and diffusible pigments acted like a pH indicator and changed light reddish yellow to dark yellowish brown by the addition of 0.1N HCl. The macroscopic properties of strain AA0851 on various agar media were summarized in Table 1.

**Physiological characteristics**

The physiological characteristics and the utilization of carbon sources were shown in Tables 2 and 3, respectively.

Aerobic actinomycetes which forms a branched substrate mycelium with a sparse to abundant aerial mycelium, sometimes forming short chains of arthrospores on an aerial mycelium and having a wall chemotype III-B and a type PI phospholipid pattern, are classified as the genus Actinomadura by Lechevalier and Lechevalier (Lechevalier, H. A. & Lechevalier, M. P.: A critical evaluation of the genera of aerobic actinomycetes. In The Actinomycetatales, pp. 393-405. Jena: Gustav Fischer Verlag. 1970). According to Fischer et al. (Fischer, A., R. N. Kroppenstedt and E. Stackebrandt: Molecular genetic and chemotaxonomic studies on Actinomadura and Nocardiopsis, J. Gen. Microbiol. 130: 817-823, 1984) and Goodfellow (Goodfellow, M., E. Stackebrandt and R. M. Kroppenstedt: Chemotaxonomy and actinomycete systematics. In Biology of Actinomycetes '88 pp. 233-238, 1988), Actinomadura is markedly heterogeneous and species of this genus may further be divided into two groups, i.e., Actinomadura madurae group and Actinomadurae pusilla group based on the phospholipid pattern, predominant menaquinone composition and fatty acid type. According to their criteria, strain AA0851 was considered to represent a Actinomadura madurae-group strain.

Comparison of strain AA0851 with recognized species belonging to A. madurae group indicated that there were no descriptions of species characterized by white to light gray aerial massed of short chains of less than 20 spores with spiny and by the formation of reddish purple diffusible pigments which was a pH indicator.

Although, Microbispora echinospora as reported to be closely related to Actinomadura based on its cell wall chemotaxonomy (Poschner, J., R. M. Kroppenstedt, A. Fischer, and E. Stackebrandt: DNA, DNA reassociation and chemotaxonomic studies on Actinomadura, Microbiospora, Microtetraspora, Micropolyspora and Nocardiopsis. Syst. Appl. Microbiol. 6: 264-270, 1986), strain AA0851 was clearly distinguished from this species on the basis of differences in spore chain morphology, cultural characteristics as well as utilization of carbon sources. Thus, strain AA0851 was identified as a species of Actinomadura.

Strain AA0851 was deposited with the American Type Culture Collection, 12301 Parklawn Drive,

Rockville, Maryland on January 3, 1991. The culture was accepted for deposit under the Budapest Treaty on the International Recognition of the Deposit of Microorganism for the Purpose of Patent Procedure. Strain AA0851 has the accession number ATCC 55138.

Table 1. Cultural characteristics of Strain AA0851

| Medium | Growth | Reverse | Aerial mycelium | Soluble pigment |
|---|---|---|---|---|
| Sucrose nitrate agar (Waksman med. No.1) | Good Purplish pink (8) | Purplish pink (8) | None | Colorless - pale yellowish pink (6) |
| Glycerol nitrate agar | Good Pale reddish yellow (130) | Pale reddish yellow (130) | None | Purplish pink (16) |
| Glucose asparagine agar (Waksman med. No. 2) | Good Light brown (90) | Light brown (90) | None | Pale yellowish pink (7) |
| Yeast ext.-malt ext. agar (ISP med. No. 2) | Good Dark red (57) | Dark red (57) | Pinkish white (391), Scant | Dark red (57) |
| Oat meal agar (ISP med. No. 3) | Good Pale pink (5) | Pale pink (5) | White (389) Scant | Pale pink (5) |
| Inorganic salts-starch agar (ISP med. No. 4) | Moderate Grayish pink (31) | Grayish pink (31) | Pinkish white (391)-light gray (398), Powdery | Deep pink (22) |
| Glycerol asparagine agar (ISP med. No. 5) | Scant, Pale reddish yellow (125) | Pale reddish yellow (125) | White (388) Scant | Pink (12) |
| Tyrosine agar (ISP med. No. 7) | Scant, Pale reddish yellow (125) | Pale reddish yellow (125) | White (391) Scant | None |
| Nutrient agar (Waksman med. No. 14) | Moderate Grayish pink (30) | Grayish pink (30) | White (389) Powdery, Scant | Grayish pink (30) |
| Yeast starch agar | Good Dark red (57) | Dark red (57) | Grayish white (390) Powdery, Good | Dark red (57) |

Table 2. Physiological characteristics of strain AA0851

| Test | Results |
|---|---|
| Starch hydrolysis (ISP med. No. 4) | Negative |
| Nitrate reduction (Difco, nitrate broth) | Positive |
| 10% skimmed milk (Difco, 10% skimmed milk) | |
| Coagulation | Positive |
| Peptonization | Positive |
| Cellulose decomposition (sucrose nitrate solution with a strip of paper as the sole carbon source) | Negative<br>Good growth |
| Gelatin liquefaction | |
| On plain gelatin | Doubtful |
| On glucose peptone gelatin | Positive |
| Melanin formation | |
| On ISP med. No. 7 | Negative |
| Vitamin B requirement | Negative |
| Temperature range for growth (°C) | 21 - 45 |
| Optimum temperature (°C) (On yeast starch agar) | 30 - 40 |
| pH range for growth | 6 - 9 |
| Optimum pH (On trypticase soy broth, BBL) | 8 |
| GC% | 73.1% |

Table 3

| Utilization of carbon sources by strain AA0851 | |
|---|---|
| Carbon source | Utilization |
| D-Glucose | + |
| L-Arabinose | + |
| D-Xylose | + |
| Inositol | - |
| Mannitol | - |
| D-Fructose | + |
| L-Rhamnose | + + |
| Sucrose | + |
| Raffinose | + + |
| - : Negative<br>+ : Weak positive<br>+ + : Strong positive<br>(ISP med. No. 9, 28 °C for 2 weeks) | |

## PRODUCTION OF PRADIMICINS S AND FS

### Fermentation

The general procedures which have been used for the fermentation of other antibiotics from actinomycetes are applicable to the present invention. For example, pradimicins S and FS may be produced by cultivating the pradimicins S and FS-producing strain of Actinomadura sp. AA0851 or a mutant or variant thereof, under a submerged aerobic condition in an aqueous nutrient medium.

The nutrient medium contains an assimilable carbon source, for example, sucrose, lactose, glucose, rhamnose, fructose, mannose, melibiose, glycerol or soluble starch to name a few. Optimal production of pradimicin S was observed when sucrose, glucose or sucrose plus glucose was used as a carbon source.

The nutrient medium should also contain an assimilable nitrogen source such as fish meal, peptone, soybean flour, peanut meal, cottonseed meal, corn steep liquor, yeast extract or ammonium salts.

As has been reported in other many cases, the production of pradimicin S is also influenced by the concentration of certain minerals present in the media. In particular, the effect of sulfur compounds on the pradimicin S production was worth mentioning, since pradimicin S possesses a sulfate group in the molecule. Among several sulfur compounds tested for increasing the production of pradimicin S, such as magnesium sulfate, calcium sulfate, ferrous sulfate, ferric sulfate, sodium sulfate, sodium sulfite, ammonium sulfate, L-methionine, L-cysteine, homocysteine, taurine, and sodium thiosulfate, either ferrous or ferric sulfate was most effective. This result indicates that iron plays an important role in the biosynthesis of pradimicin S. This hypothesis is supported by the observation that the pradimicin S production was increased in the presence of both ferric chloride and zinc sulfate. The optimum concentration of ferrous sulfate ranged from 0.1 to 0.4% and that of ferric sulfate was 0.1%.

Other inorganic salts such as sodium chloride, potassium chloride, calcium carbonate, phosphates, etc. are added if necessary. Trace elements such as copper, manganese, etc. are added to the medium if required, or they may be supplied as impurities of other constituents of the medium.

Production of pradimicins S and FS can be effected at any temperature conducive to satisfactory growth of the producing organism, e.g. 15-45 °C, but it is preferable to conduct the fermentation at 21-45 °C, and most preferably at 25-32 °C. A neutral pH is preferably employed in the medium and the production of the antibiotics is carried out generally for a period of about five to fourteen days.

The fermentation may be carried out in flasks or in laboratory or industrial fermentors of various capacities. When tank fermentation is to be used, it is desirable to produce a vegetative inoculum in a nutrient broth by inoculating a small volume of the culture medium with a slant or soil culture or a lyophilized culture of the organism. After obtaining an active inoculum in this manner, it is transferred aseptically to the fermentation medium for large scale production of the antibiotic. The medium in which the vegetative inoculum is produced can be the same as, or different from that utilized in the tank as long as it

is such that a good growth of the producing organism is obtained. Agitation during the fermentation can be provided by a mechanical impeller and conventional antifoam agents such as lard oil or silicon oil can be added if needed.

## Isolation and purification

Production of the antibiotics in the fermentation medium can be readily followed during the course of the fermentation by conventional techniques such as thin layer chromatography, HPLC, and bioassays.

For the purpose of the isolation and purification of pradimicins S and FS, usual procedures for hydrophilic acidic substances, such as organic solvent extraction, ion exchange resin, partition chromatography, acidic precipitation, etc., may be used either alone or in combination. For example, the following isolation procedure for pradimicin S is illustrative of the present invention.

An entire fermentation broth is separated into a mycelial cake and a supernatant by a conventional method such as centrifugation. Pradimicin S in the supernatant is adsorbed to high porous polymer resin such as Diaion HP-20 (Mitsubishi Kasei Co.) and eluted with a water-miscible organic solvent such as acetone. After the eluates are evaporated, the residue is dissolved in a mixture of $CH_3CN$-0.15% $KH_2PO_4$, pH 3.5 solution and applied to a reversed phase silica gel column such as YMC-ODS A60 (Yamamura Chemical Lab.). The column is eluted with the same solvent and the active fractions are concentrated to remove $CH_3CN$. The phosphate is removed by Diaion HP-20. The eluates are concentrated and lyophilized to give semi-pure pradimicin S hydrochloride. For further purification, the above-described semi-pure pradimicin S hydrochloride is subjected to a column of reversed phase silica gel such as Lichroprep® RP-18 (Merck Corp.). The fractions containing pradimicin S are collected and desalted to provide pure pradimicin S hydrochloride.

Analogous procedures of fermentation, isolation and purification can be used to make pradimicin FS.

## PHYSICO-CHEMICAL PROPERTIES AND STRUCTURES

### Pradimicin S

Pradimicin S is an acidic antibiotic. Disodium salt of pradimicin S (Pradimicin S•2Na) was isolated as dark red needles and possesses physico-chemical properties as shown in Table 4. This antibiotic can be clearly distinguished from the other known pradimicins, including pradimicin L, by difference in the TLC and HPLC mobilities. Pradimicin S hydrochloride (pradimicin S•HCl) is soluble in dimethyl sulfoxide, dimethylformamide and alkaline water, and slightly soluble in ethanol, methanol, acetone and water, but practically insoluble in acidic water and other common organic solvents. Pradimicin S•2Na shows good solubility in water. The IR spectrum of Pradimicin S•2Na in KBr showed absorptions at 3,400, 1,620, 1,605, 1,260 and 1,070 $cm^{-1}$, while the acidic form of pradimicin S exhibited a carbonyl absorption band at 1,718 $cm^{-1}$ (Fig. 1), indicating the presence of a carboxyl group in the structure of pradimicin S. The molecular formula of pradimicin S was determined to be $C_{41}H_{46}N_2O_{22}S$ based on its FAB-MS (positive, m/z 951 $(M+H)^+$ and negative, m/z 949 $(M-H)^-$) and HR-FAB-MS (positive, m/z 951.2323 $(M+H)^+$). This pseudo-molecular ion peak at m/z 951 $(M+H)^+$ differs from that of pradimicin L (m/z 871 $(M+H)^+$) by 80 mass units. Moreover, FAB-MS (negative) of pradimicin S displayed characteristic fragment ions (m/z 80 and 97) of the sulfate which were not detected in FAB-MS (positive).

Further structural information of pradimicin S was obtained by its carbon and proton nuclear magnetic resonance spectral analyses (Figs. 2 and 3) and additional degradation studies.

Table 4

| Physico-chemical properties of pradimicin S Na salt | |
|---|---|
| Nature | Dark red needles |
| M.P. (dec.) | >180°C |
| $[\alpha]_D^{26}$ | +400° (C 0.02, $H_2O$) |
| FAB-MS (positive) m/z | 951 $(M+H)^+$, 973 $(M+Na)^+$ |
| FAB-MS (negative) m/z | 949 $(M\text{-}H)^-$, 972 $(M\text{-}H+Na)^-$ |
| HR-FAB-MS, $(M+H)^+$ m/z | 951.2323 |
| Molecular formula | $C_{41}H_{44}N_2O_{22}S \cdot Na_2$ |
| UV $\lambda_{max}$ nm ($\epsilon$) in 0.02N NaOH-MeOH (1:1) | 243 (34,900), 319 (15,900), 498 (16,000) |
| in 0.02N HCl-MeOH (1:1) | 234 (33,300), 300 (30,000), 460 (12,600) |
| IR $\nu_{max}$ (KBr) $cm^{-1}$ | 3,400, 1,620, 1,605, 1,440, 1,385, 1,260, 1,160, 1,070 |
| TLC ($SiO_2$), Rf | 0.25 |
| (MeOAc-n-PrOH-28% $NH_4OH$ = 45 : 105 : 60 V/V) | |
| HPLC Rt (min.) | 7.22 |
| (ODS, $CH_3CN$-0.15% $KH_2PO_4$, pH 3.5 = 30 : 70, V/V) | |

**Pradimicin FS**

Pradimicin FS is an acidic antibiotic. Disodium salt of pradimicin FS (pradimicin $FS \cdot Na_2$) possesses physico-chemical properties as shown in Table 5. Pradimicin $FS \cdot Na_2$ is soluble in dimethyl sulfoxide, dimethylformamide and water, slightly soluble in ethanol, methanol and acetone, but practically insoluble in acidic water and other common organic solvents such as ethyl acetate, benzene and chloroform. Pradimicin $FS \cdot Na_2$ shows good solubility in water. The molecular formula of pradimicin FS was determined to be $C_{41}H_{46}N_2O_{23}S$ based on its FAB-MS (positive, m/z 967 $(M + H)^+$). This pseudo-molecular ion peak differs from that of pradimicin S (m/z 951 $(M + H)^+$) by 16 mass units. Further structural information on pradimicin FS was obtained from its carbon and proton nuclear magnetic resonance spectral analyses (Figs. 6 and 7).

Table 5

| Physico-chemical properties of Pradimicin FS | |
|---|---|
| Nature | amorphous violet powder |
| M.P. (dec.) | > 180°C |
| FAB-MS (positive) m/z | 967 $(M+H)^+$, 989 $(M+Na)^+$ |
| Molecular formula UV $\lambda_{max}$ nm ($\epsilon$) | $C_{41}H_{44}N_2O_{23}S \cdot Na_2$ |
| in 0.02N NaOH-MeOH (1:1) | 240(34,900), 320(15,600), 498 (14,500) |
| IR $\nu_{max}$ (KBr) $cm^{-1}$ | 3410, 1625, 1610, 1445, 1390, 1260, 1160, 1060 |
| HPLC Rt (min.)* | 6.2 |

* : ODS, $CH_3CN$/0.02M phosphate buffer, pH 7.0 (15/85, v/v)

**DESCRIPTION OF SPECIFIC EMBODIMENTS**

The following specific embodiments are intended to be merely illustrative and are not intended to limit the scope of the invention. For example, it is not the intention of this invention to limit the use to the particular Actinomadura sp. AA0851 or to organisms fully answering the above description for the production of pradimicins S and FS. Rather, this invention includes other pradimicins S and FS producing strains or mutants or variants of said organism which can be produced from the described organism by known means such as X-ray radiation, ultraviolet radiation, treatment with nitrogen mustard, phage exposure, and the like.

Except otherwise noted, v/v refers to volume/volume.

**PRADIMICIN S**

**Example 1. Seed culture**

Actinomadura sp AA0851 was propagated on 1/2 ISP-4 medium. After incubation at 30 ° C for 3 weeks, a portion of this agar slant was inoculated into a 500-ml Erlenmeyer flask containing 100 ml of tripticase soy broth (BBL, Becton Dickinson and Co., Cockeysville, MD) and incubated at 32 ° C for 7 days on a rotary shaker orbiting at 200 rpm. The resulting vegetative mycelia were washed and resuspended with half volume of 20% glycerol, and then stored at -80 ° C. The seed culture was prepared by inoculating 1 ml of these freezed culture into 100 ml of tripticase soy broth in a 500-ml Erlenmeyer flask and incubating for 4 days at 32 ° C.

**Example 2. Flask fermentation**

Each 5-ml portion of the seed as set forth in Example 1 was transferred into 500-ml of Erlenmeyer flasks containing 100 ml of production medium (designated 164 medium) composed of soluble starch 2.0%, glucose 1.0%, Pharmamedia (Traders Protein) 1.0%, Brewer's yeast extract (Kirin Brewery Co., Ltd.) 0.3%, NZ-amine (Scheffield Products) 0.3%, $CaCO_3$ 0.1% and Allophane (Shinagawa Brick) 0.5% (pH adjusted to 7.5 before autoclaving). The fermentation was carried out for 7 days at 28 ° C on a rotary shaker. The progress of fermentation was monitored by judging from the visible absorption of an aliquot taken periodically at 500 nm in 0.02N NaOH-MeOH (1:1) solution. For quantitative analysis of different pradimicin components, the supernatant of broth was diluted ten fold with DMSO, and then filtered through a membrane filter (Gelman Sciences Japan, Ltd., Ekicrodisc 13CR, Pore size: 0.45 μm). This filtrate (10 μl) was analyzed by HPLC on Excel pak SIL-$C_{18}$5R (Yokogawa Electronic Co., Ltd.) using acetonitrile-0.15% $KH_2PO_4$ buffer, pH 3.5 (27:73), at a flow rate of 1 ml/min with 460 nm detection. The fermentation yield of pradimicins in the 164 medium was 120 μg/ml (Pradimicin S: 60 μg/ml, Pradimicin B: 40 μg/ml, Pradimicin L: 9 μg/ml).

**Example 3. Alternative flask fermentation**

Each 5-ml portion of the seed culture described in Example 1 was transferred into 500-ml of Erlenmeyer flasks containing 100 ml of a production medium (designated FR20-1) composed of sucrose 3.0%, glucose 1.0%, Pharmamedia 3.0%, $FeSO_4 \cdot 7H_2O$ 0.1%, $CaCO_3$ 0.3% (pH adjusted to 7.5 before autoclaving). Fermentation was conducted on a rotary shaker operating at 200 rpm for 12 days at 28 ° C, in which the total yield of pradimicins was 2,258 μg/ml (Pradimicin S: 1,242 μg/ml, Pradimicin B: 693 μg/ml and Pradimicin L: 136 μg/ml).

**Example 4. Isolation of pradimicin S by solvent extraction method**

The fermentation broth (7.0 L) as set forth in Example 2 was centrifuged. The supernatant (10 L) was extracted with n-BuOH (3 L) at pH 2.0 adjusted with 6N HCl. The n-BuOH layer was back-extracted with alkaline water (pH 8.5, 1 L) adjusted with 6N NaOH. The water layer was concentrated in vacuo to remove n-BuOH and then acidified to pH 2.0 using 6N HCl. The precipitate formed was collected by filtration and then dissolved in 400 ml of water adjusted to pH 8.0 with 6N NaOH. The solution was applied onto a column of Diaion HP-20 (2 L, Mitsubishi Kasei Co.). The column was washed with water (10 L) and the products eluted with acetone (1.4 L). The eluate was concentrated in vacuo to 950 ml and then washed twice with EtOAc (400 ml) at pH 2.0 to remove impurities. The precipitate formed was collected by filtration and lyophilized from water (90 ml), after being adjusted to pH 8.0 with 6N NaOH, to yield 790 mg of crude solid. The solid (780 mg) was dissolved in 125 ml of a mixture of $CH_3CN$-0.15% $KH_2PO_4$, pH 3.5 (24:76) and subjected to chromatography on a column of YMC gel, ODS-A60 (5 L, Yamamura Chemical Lab.) which had been equilibrated with the same solvent system. Elution was done with the same solvent, and the eluates were monitored by HPLC (Column: YMC A301-3, 4.6 mm I.D. x 100 mm, 3 μm, Yamamura Chemical Lab., Mobile phase: $CH_3CN$-0.15% $KH_2PO_4$, pH 3.5 (3:7), Flow rate: 1.0 ml/min., Detection: UV absorption at 460 nm, Retention time: pradimicin S, 7.22 min). The relevant fractions were concentrated, desalted by Diaion HP-20 to yield pure pradimicin S (161 mg). This powder (132 mg) was crystallized using a solvent mixture of 1/10N NaOH (28 ml), EtOH (56 ml) and EtOAc (10 ml) to give 97 mg of pure pradimicin S sodium salt as the dark red needles.

**Example 5. An alternative isolation method of pradimicin S**

The whole broth (11.5 L) as set forth in Example 3 was centrifuged. Pradimicin S in the supernatant (14 L) was adsorbed to 3.5 L of Diaion HP-20, and then eluted with 2.5 L of 80% aq. acetone after the resin was washed with water. The eluates were concentrated and then lyophilized to yield 28 g of a crude solid. After the crude solid was dissolved in 3.8 L of a mixture of $CH_3CN$-0.15% $KH_2PO_4$, pH 3.5 (21:79), pradimicin S in this solution was adsorbed onto 1.0 L of reversed phase silica gel, YMC-A60 and then applied onto a column of the same adsorbent (10L) which was pre-equilibrated with a mixture of $CH_3CN$-0.15% $KH_2PO_4$, pH 3.5 (22.5:77.5). The column was eluted with the same solvent system, and the eluates were monitored by HPLC. The relevant fractions (120 L) were concentrated, applied to a column of Diaion HP-20 (2.4 L) for desalting, and then eluted with 60% aq. acetone after the resin was washed with water. Active fractions (1.5 L) were concentrated and lyophilized to yield 8 g of semi-pure pradimicin S hydrochloride.

The semi-pure pradimicin S (1.83 g) was dissolved in 340 ml of $CH_3CN$-0.15% $KH_2PO_4$, pH 3.5 (18:82) which was further purified on a column of Lichroprep® RP-18 (4 L) being eluted with $CH_3CN$-0.15% $KH_2PO_4$, pH 3.5 (24:76). The active fractions were concentrated and desalted by a Diaion HP-20 column (800 ml) with 80% aq. acetone (400 ml) as the eluant after a column was washed with water. The eluates were concentrated and lyophilized to afford 1.5 g of pure pradimicin S. The pure powder was crystallized using 800 ml of a solvent mixture of 1/10N NaOH-EtOH-EtOAc (2:4:0.7) to give 1.2 g of pure pradimicin S sodium salt as the dark red needles.

**Example 6 Production of N-methyl Pradicimin S (BMY-29273)**

To pradimicin S (20 mg, sodium salt) in water (2 ml)-acetonitrile (2 ml) was added formaldehyde (> 35%, 0.2 ml) and sodium cyanoborohydride (30 mg). The solution was allowed to stand for 19 hours at room temperature, and the progress of reaction was monitored by HPLC. The reaction solution was diluted with water (26 ml). This solution was applied onto a column of Diaion HP-20 (20 ml). The column was washed with water (50 ml) and eluted with 80% aqueous acetone (20 ml). Concentration of the dark-red eluate afforded amorphous powder of N-methyl pradimicin S sodium salt (17.8 mg). Physico-chemcial properties of this derivative are summarized in Table 6 below.

Table 6

| Physico-chemical properties of N-methyl pradimicin S (BMY-29273) sodium salt | |
|---|---|
| Nature | amorphous violet powder |
| M.P. (dec.) | > 195°C |
| FAB-MS (negative) m/z | 963 $(M-H)^-$ |
| Molecular formula | $C_{42}H_{46}N_2O_{22}S \cdot Na_2$ |
| UV $\lambda_{max}$ nm ($\epsilon$) in 0.02N NaOH-MeOH (1:1) | 319 (14,000), 500 (13,200) |
| IR $\nu_{max}$ (KBr) $cm^{-1}$ | 3400, 1625, 1590, 1450, 1385, 1260, 1070 |
| $^1H$-NMR (400 MHz, DMSO-$\underline{d}_6$) | $\delta$ 2.59 (6H, s, $N(CH_3)_2$) |
| HPLC Rt (min.)* | 17.00 |

* : ODS, $CH_3CN$/0.15% $KH_2PO_4$, pH3.5 (25/75, v/v)

**PRADIMICIN FS**

**Example 7. Flask Fermentation**

Agar slant of Actinomadura sp. AA 0851 was inoculated into a 500 ml Erlenmeyer flask containing 100 ml of seed medium composed of sucrose 1%, Pharmamedia 0.5%, yeast extract (Oriental yeast) 0.5% and $CaCO_3$ 0.1% (pH adjusted to 7.0 before autoclaving), and then incubated at 32°C for 5 days on a rotary shaker orbiting at 200 rpm. Each 5-ml portion of the seed culture was transferred into 500-ml of Erlenmeyer flasks containing 100 ml of a production medium (designated FR-21 medium) composed of sucrose 3%, glucose 1%, Pharmamedia 3%, $FeSO_4 \cdot 7H_2O$ 0.1%, $CaCO_3$ 0.3%, D-serine 0.25% and D-cycloserine 0.001% (pH adjusted to 7.5 before autoclaving). The fermentation was carried out for 13 days at 28°C on a rotary shaker. The progress of fermentation was monitored by judging from the visible absorption of an

aliquot taken periodically at 500 nm in 0.02 N NaOH-MeOH(1:1) solution. For quantitative analysis of different pradimicin components, the supernatant of broth was diluted ten fold with DMSO, and then filtered through a membrane filter (Gelman Sciences Japan, Ltd., Ekicrodisc 13CR, Pore size: 0.45 µm). This filtrate (10 µl) was analyzed by HPLC on Excel pak SIL-$C_{18}$5R (Yokogawa Electronic Co., Ltd.) using acetonitrile-0.15% $KH_2PO_4$ buffer, pH 3.5 (27:73), at a flow rate of 1 ml/minute with 460 nm detection. The fermentation yield of pradimicins in the FR-21 medium was 900 µg/ml (pradimicin FS: 290 µg/ml, pradimicin S: 234 µg/ml, pradimicin B: 171 µg/ml and pradimicin FB (desxylosylpradimicin FA-1, BMY-28944): 144 µg/ml).

**Example 8. Isolation and purification**

The whole broth (9 L) was centrifuged. Pradimicin FS in the supernatant (10 L) was adsorbed to 3.0 L of Diaion HP-20 (Mitsubishi Kasei Co.) and then eluted with 2.6 L of 80% aq. acetone after the resin was washed with water (50 L). The eluates were concentrated and then lyophilized to yield 17.5 g of crude solid. The solid (17 g) was dissolved in 2.0 L of water adjusted to pH 7.5 with 0.1 NaOH, and then filtered to remove insoluble impurities. The solution was adjusted to pH 2.5 with 0.1 N HCl to give precipitate containing pradimicin FS as a major component. The precipitate formed was collected by filtration and washed with 0.001 N HCl (1.5 L). Moreover, the precipitate was dissolved in 150 ml of water adjusted to pH 7.6 with 1N NaOH and this solution was dropped into acetone (1.5 L). The precipitate deposited was washed with acetone (600 ml) and lyophilized from water (350 ml) after adjusting to pH 7.0 with 0.1 N HCl to yield 7.2 g of partially purified sample. The solid (2.5 g) was dissolved in 250 ml of a mixture of $CH_3CN$-0.02 M phosphate buffer, pH 7.0 (15:85) and subjected to chromatography on a column of YMC gel, ODS-A60 (5 L, Yamaura Chemical Lab.) which had been equilibrated with the same solvent system. Elution was done with the same solvent, and the eluates were monitored by HPLC (Column: Cosmosil 5$C_{18}$-AR, 5 µm, 4.6 mm I.D. x 150 mm, Nacalai Tesque Inc., Mobile phase: $CH_3CN$-0.02M phosphate buffer, pH 7.0 (15:85), Flow rate: 1.0 ml/min., Detection: UV absorption at 254 nm, Retention time: pradimicin FS, 6.2 min.). The relevant fractions (7.7 L) were concentrated, applied to a column of Diaion HP-20 (250 ml) with the eluant of 60% aq. acetone (350 ml) after the column was washed with 0.001 N HCl (1.0L). The eluates were concentrated and lyophilized from water (40 ml) after adjusting to pH 5.0 with 0.1 N NaOH to afford 520 mg of semi-pure pradimicin FS. To remove contaminated inorganic salts, an aqueous solution (260 ml) of the sample (500 mg) was adjusted to pH 2.9 with 0.1 N HCl to deposit HCl salt of pure pradimicin FS (409 mg). In order to convert the HCl salt to its sodium salt, the HCl salt (250 mg) was lyophilized from water (40 ml) after adjusted to pH 7.5 with 0.1 NaOH to afford 257 mg of homogeneous pradimicin FS•disodium salt.

**BIOLOGICAL ACTIVITIES**

**Antifungal activity**

The in vitro antifungal activity of pradimicins was determined by a conventional agar dilution method using Yeast Morphology agar containing 1/15 M phosphate buffer (pH 7.0). The results are summarized in Table 7. Pradimicins S and FS and N-methyl pradimicin S (BMY-29273) were found to have activity against various fungal organisms.

The in vivo efficacy of pradimicins was evaluated against C. albicans A9540 systemic infection in male ICR mice (20-24 g body weight). The pathogen was cultured for 18 hours at 28°C in YGP medium (yeast extract 0.2%, glucose 1.5%, peptone 0.5%, $K_2HPO_4$ 0.05% and $MgSO_4$ 0.05%) and resuspended in saline. Mice were challenged intravenously with 10 times lethal dose of the pathogen. Immediately after the fungal challenge, groups of 5 mice received a single intravenous injection of pradimicin S or FS or N-methyl pradimicin S at various dose levels. Amphotericin B and Fluconazol were used as positive controls. The median protective dose ($PD_{50}$) was determined from the number of surviving mice recorded on the 20th day after the fungal infection. The results are shown in Table 8.

Table 7. In vitro antifungal activity

| Test organism | MIC (μg/ml) | | | |
|---|---|---|---|---|
| | Pradimicin S/FS | BMY-29273 | Amphotericin B | Ketoconazole |
| Saccharomyces cerevisiae ATCC 9763 | 3.1 / 6.3 | 3.1 | 0.2 | 100 |
| Candida albicans A9540 | 25 / 12.5 | 12.5 | 0.4 | 25 |
| Candida albicans ATCC 38247 | 6.3 / 6.3 | 6.3 | 50 | 6.3 |
| Candida albicans ATCC 32354 (B311) | 6.3 / 12.5 | 12.5 | 0.2 | 50 |
| Candida albicans 83-2-14 (Juntendo) | 25 / 12.5 | 12.5 | 0.4 | 25 |
| Candida tropicalis 85-8 (Kitasato) | 50 / 25 | 50 | 0.4 | 100 |
| Candida tropicalis IFO 10241 | 50 / 25 | 50 | 0.4 | 50 |
| Cryptococcus neoformans D49 | 1.6 / 6.3 | 12.5 | 0.4 | 6.3 |
| Cryptococcus sp. IAM 4514 | 1.6 / 6.3 | 12.5 | 0.4 | 6.3 |
| Aspergillus fumigatus IAM 2034 | 3.1 / 6.3 | >100 | 0.4 | 3.1 |
| Trichophyton mentagrophytes #4329 | 6.3 / 6.3 | 12.5 | 0.4 | 0.8 |

Medium : Yeast Morphology agar + 1/15M phosphate buffer (pH 7.0)
Inoculum Size : $10^6$ cells/ml (except T. mentagrophytes: $10^7$ cells/ml)
Incubation conditions : 28°C, 40 hrs. (60 hrs. for T. mentagrophytes)

Table 8

| In vivo activity against C. albicans A9540 systemic infection in mice | |
|---|---|
| Compound | $PD_{50}$ mg/kg (i.v.) |
| Pradimicin S | 35 |
| Pradimicin FS | 18 |
| BMY-29273 | 47 |
| Amphotericin B | 0.31 |
| Fluconazol | >50 |

**Antiviral activity**

The in vitro antiviral activity of pradimicins S and FS was assessed using the herpes simplex virus type I (HSV-1)-Vero cell and influenza virus A-Madin Darby canine kidney (MDCK) cell systems by the dye-uptake assay (Antiviral Research 3, p. 223-234, 1986). A 200 µl aliquot of cell suspension containing 2 x $10^4$ cells was inoculated to each well of 96-well microplates and cultured at 37°C for 48-72 hours under humidified 5% $CO_2$-95% air. Thereafter, the growth medium was replaced by 250 µl of a fresh medium containing a test compound, to which a 50 µl medium containing approximately 10 x $TCID_{50}$ of a virus was added. After 72 hours of incubation, the degree of inhibition of viral-induced cytopathic effect and the drug-induced cytotoxicity were determined. $ID_{50}$ was expressed as the concentration showing 50% inhibition of the cytopathic effect compared to that of control and $TD_{50}$ was the concentration exhibiting 50% cytotoxicity against the Vero or MDCK cells without a viral infection. Acyclovir and ribavirin were used as reference compounds of anti-HSV activity and anti-influenza virus A activity, respectively. The results are shown in Table 9.

Table 9

| Antiviral activity against herpes simplex virus type I and influenza virus A | | | | |
|---|---|---|---|---|
| | HSV-Vero cell | | Influenza virus-MDCK cell | |
| | $ID_{50}$(µg/ml) | $ID_{50}$(µg/ml) | $ID_{50}$(µg/ml) | $ID_{50}$(µg/ml) |
| Pradimicin S | >100 | >100 | 22 | >100 |
| Pradimicin FS | >100 | >100 | 82 | >100 |
| Acyclovir | 0.09 | >100 | | |
| Ribavirin | | | 9.5 | >100 |

Anti-HIV activity

Pradimicin S was evaluated for activity against human immunodeficiency virus ($LAV_{BRU}$ strain obtained from Luc Montagnier, Institute Pasteur, Paris France) in CEM-SS cells (P.L. Nara et al in AIDS Res. Human Retroviruses, 1987, 3, 283-302) using the XTT assay described by D.S. Weislow, et al in J. Natl. Cancer Institute, 1989, 81, 577-586. CEM-SS cells were obtained from Owen Weislow at the National Cancer Institute.

The antiviral effect is expressed as the concentration of a compound which increases the number of viable cells in infected cultures to 50% that of the untreated, uninfected control cultures ($ED_{50}$). The cellular toxicity is expressed as the concentration of a compound which reduces the number of viable cells to 50% that of the untreated control ($TD_{50}$). Table 10 shows the results of this assay.

Table 10

| Anti-HIV activity of Pradimicin S in CEM-SS cells evaluated by XTT assay six days post infection | | |
|---|---|---|
| Compound | $ED_{50}$ (μg/ml) | $TD_{50}$ (μg/ml) |
| Pradimicin S | 3 | 500 |
| 2', 3' -Dideoxyinosine (ddI) | 60 | >500 |

**Toxicity**

The acute toxicity of pradimicins S and FS and N-methyl pradimicin S was determined in mice after single administration (i.v.). The $LD_{50}$ value was 244 mg/kg, 210 mg/kg and ≧424 mg/kg for pradimicins S and FS and N-methyl pradimicin S, respectively.

The foregoing biological tests reveal that pradimicins of the instant invention are useful in treating mammals afflicted with fungal or viral infections. Thus, further aspect of the present invention provides a method for treating a fungal or viral infection which comprises administering to the mammal so afflicted an antifungal or antiviral effective amount of pradimicin S or FS or its N-methyl derivative.

From the above description, one skilled in the art can readily ascertain the essential characteristics of this invention, and without departing from the spirit and scope thereof, can make various changes and modifications to the invention to adapt it to various usages and conditions.

**Claims**

1. A compound of formula II or a pharmaceutically acceptable salt thereof

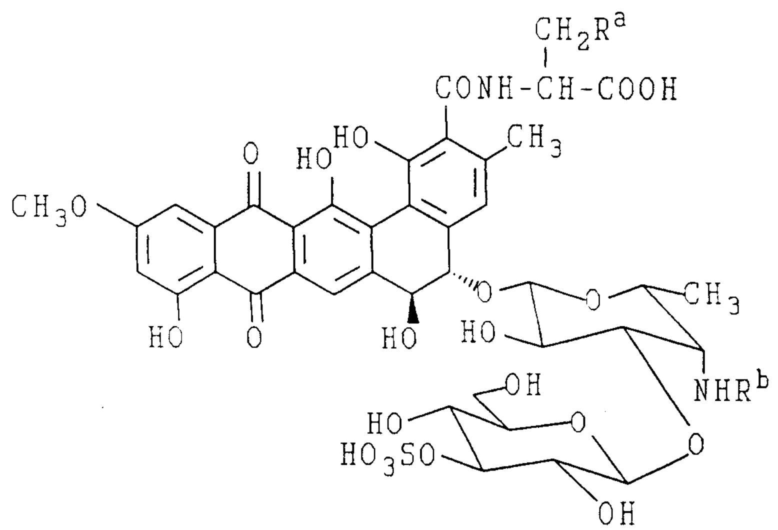

II

in which $R^a$ is hydrogen or hydroxy, $R^b$ is hydrogen or methyl, and the alanine or the serine residue has the D-configuration.

2. The compound as defined in Claim 1 in which $R^a$ is hydrogen and $R^b$ is hydrogen.

3. The compound as defined in Claim 1 in which $R^a$ is hydrogen and $R^b$ is methyl.

4. The compound as defined in Claim 1 in which $R^a$ is hydroxy and $R^b$ is hydrogen.

5. A pharmaceutical formulation which comprises as an active ingredient a compound as claimed in any one of Claims 1 to 4, or a pharmaceutically acceptable salt thereof, associated with one or more pharmaceutically acceptable carriers, excipients or diluents therefor.

6. A compound of formula II as claimed in any one of Claims 1 to 4 for use as an anti-viral or anti-fungal agent.

7. A process for the preparation of a compound as claimed in any one of Claim 1, 2, 3, or 4 which comprises culturing Actinomadura sp. AA0851 or a mutant or a variant thereof in a nutrient medium containing an assimilable source of carbon and nitrogen, or alkylating a compound as claimed in any one of Claims 1, 2 or 4 with formaldehyde and sodium cyanoborohydride.

8. The process as claimed in Claim 7 in which the nutrient medium additionally contains about 0.1 to 0.4% ferrous sulfate or about 0.1% ferric sulfate.

9. A biologically pure culture of Actinomadura sp. AA0851, ATCC No. 55138.

**Claims for the following Contracting State : ES**

1. A process for preparing a compound of formula II or a pharmaceutically acceptable salt thereof,

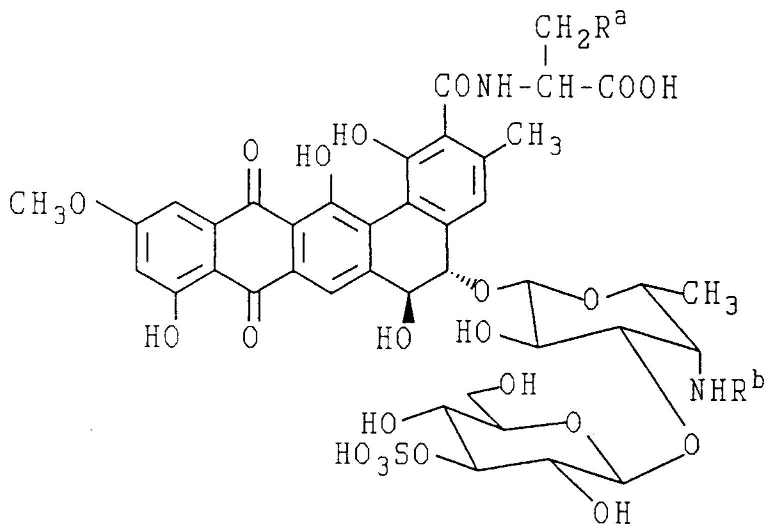

II

in which $R^a$ is hydrogen or hydroxy, $R^b$ is hydrogen or methyl, and the alanine or the serine residue has the D-configuration, which comprises:

(a) culturing Actinomadura sp. AA0851 or a mutant or a variant thereof in a nutrient medium containing an assimilable source of carbon and nitrogen; or

(b) alkylating a compound of formula II made in step (a) with formaldehyde and sodium cyanoborohydride.

2. The process as claimed in Claim 1 in which the nutrient medium additionally contains about 0.1 to 0.4% ferrous sulfate or about 0.1% ferric sulfate.

3. A process as defined in claim 1 or 2 for preparing a compound of formula II in which $R^a$ is hydrogen and $R^b$ is hydrogen.

4. A process as claimed in claim 1 for preparing a compound of formula II in which $R^a$ is hydrogen and $R^b$ is methyl.

5. A process as claimed in claim 1 or 2 for preparing a compound of formula II in which $R^a$ is hydroxy and $R^b$ is hydrogen.

6. A process for preparing a pharmaceutical formulation which comprises admixing a compound of formula II or a pharmaceutically acceptable salt thereof,

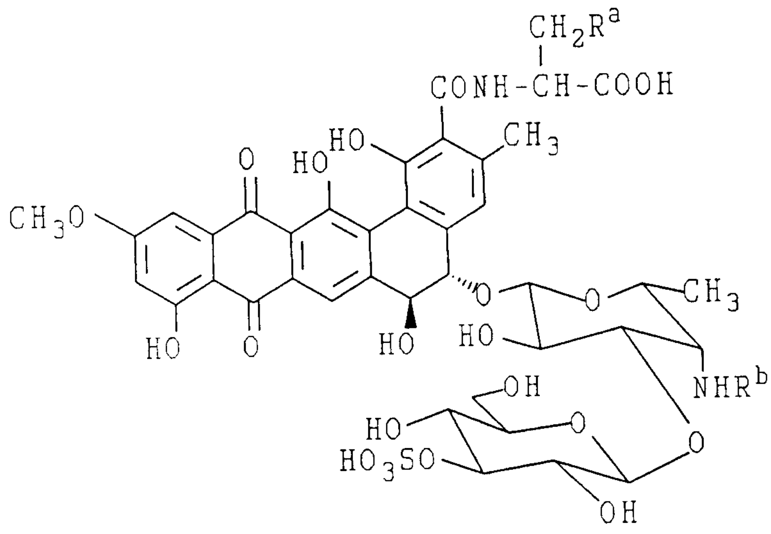

II

in which $R^a$ is hydrogen or hydroxy, $R^b$ is hydrogen or methyl, and the alanine or the serine residue has the D-configuration, with one or more pharmaceutically acceptable carriers, diluents or excipients therefor.

**7.** A biologically pure culture of Actinomadura sp. AA0851, ATCC No. 55138.

**Claims for the following Contracting State : GR**

**1.** A compound of formula II or a pharmaceutically acceptable salt thereof

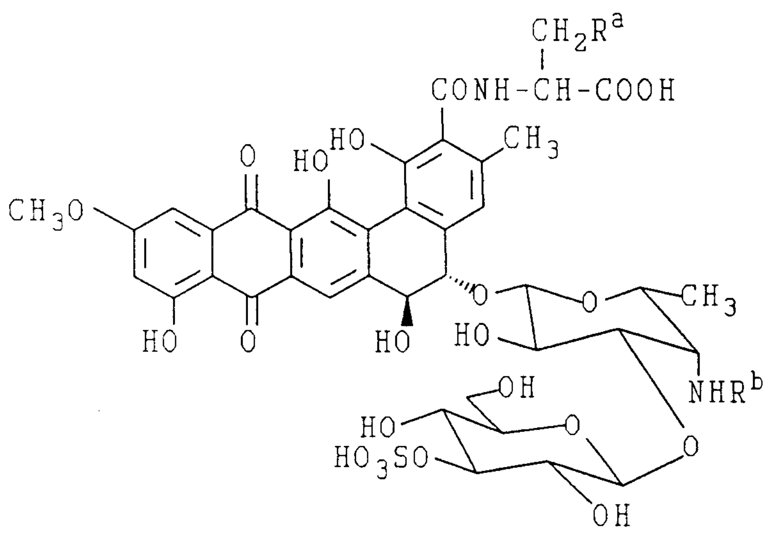

II

in which $R^a$ is hydrogen or hydroxy, $R^b$ is hydrogen or methyl, and the alanine or the serine residue has the D-configuration.

**2.** The compound as defined in Claim 1 in which $R^a$ is hydrogen and $R^b$ is hydrogen.

**3.** The compound as defined in Claim 1 in which $R^a$ is hydrogen and $R^b$ is methyl.

**4.** The compound as defined in Claim 1 in which $R^a$ is hydroxy and $R^b$ is hydrogen.

**5.** A compound of formula II as claimed in any one of Claims 1 to 4 for use as an anti-viral or anti-fungal

agent.

**6.** A process for the preparation of a compound as claimed in any one of Claim 1, 2, 3, or 4 which comprises culturing Actinomadura sp. AA0851 or a mutant or a variant thereof in a nutrient medium containing an assimilable source of carbon and nitrogen, or alkylating a compound as claimed in any one of Claims 1, 2 or 4 with formaldehyde and sodium cyanoborohydride.

**7.** The process as claimed in Claim 6 in which the nutrient medium additionally contains about 0.1 to 0.4% ferrous sulfate or about 0.1% ferric sulfate.

**8.** A process for preparing a pharmaceutical formulation which comprises admixing a compound of formula II as claimed in any one of Claims 1 to 4 with one or more pharmaceutically acceptable carriers, diluents or excipients therefor.

**9.** A biologically pure culture of Actinomadura sp. AA0851, ATCC No. 55138.

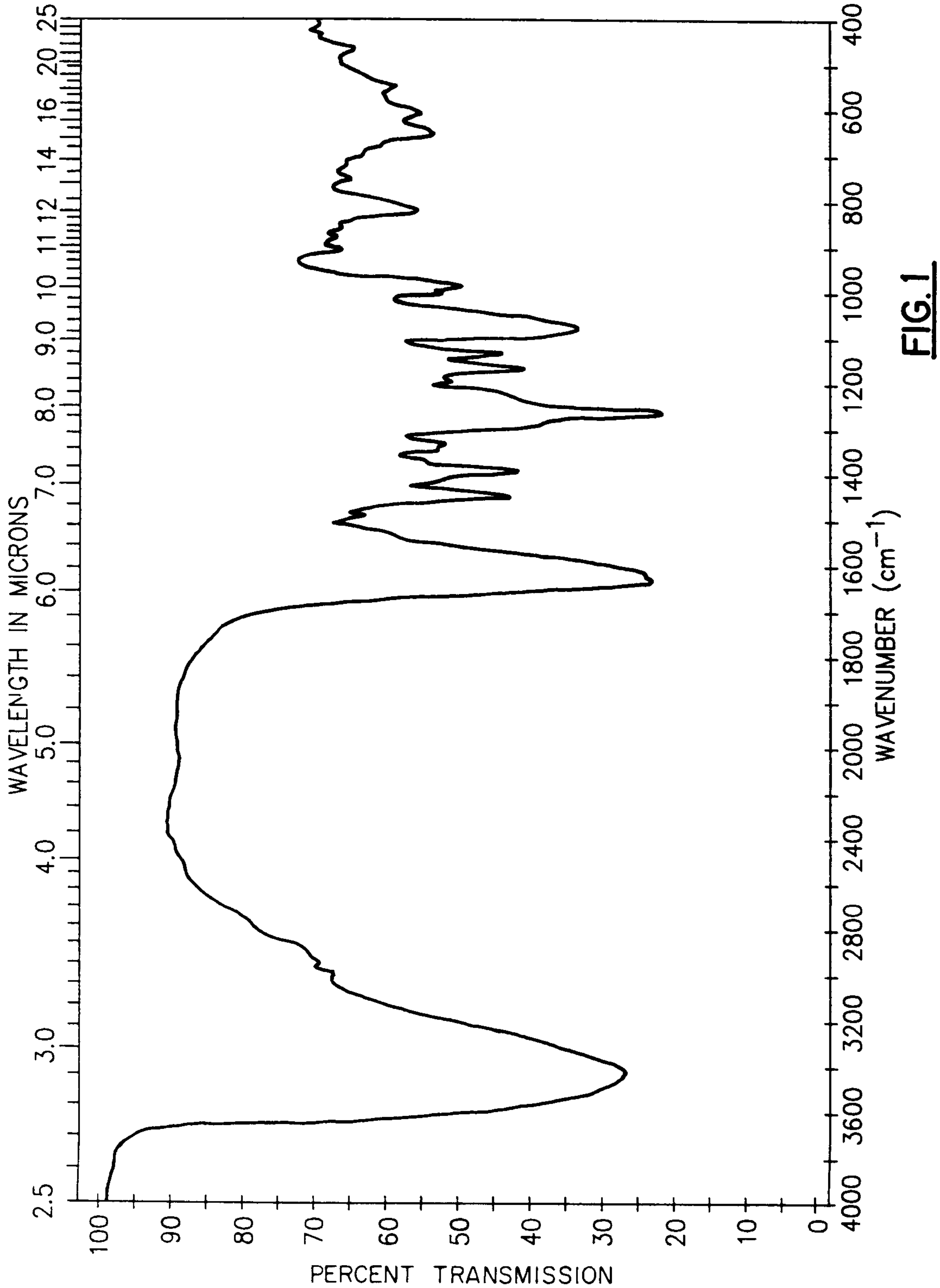
WAVELENGTH IN MICRONS
2.5
3.0
4.0
5.0
6.0
7.0
8.0
9.0
10
11 12
14
16
20
25
PERCENT TRANSMISSION
100
90
80
70
60
50
40
30
20
10
0
WAVENUMBER ($cm^{-1}$)
4000
3600
3200
2800
2400
2000
1800
1600
1400
1200
1000
800
600
400

FIG.1

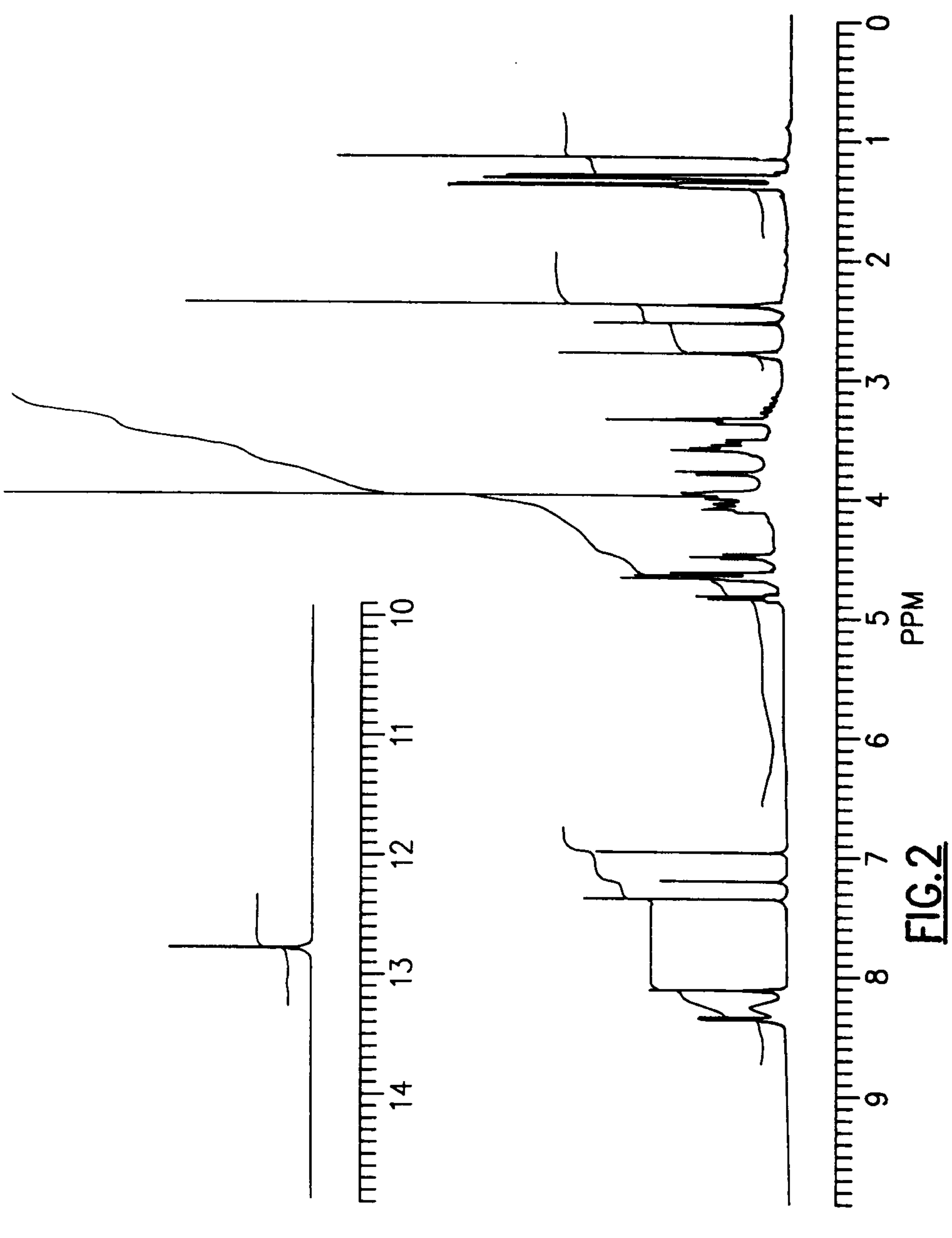


FIG.2

FIG.3

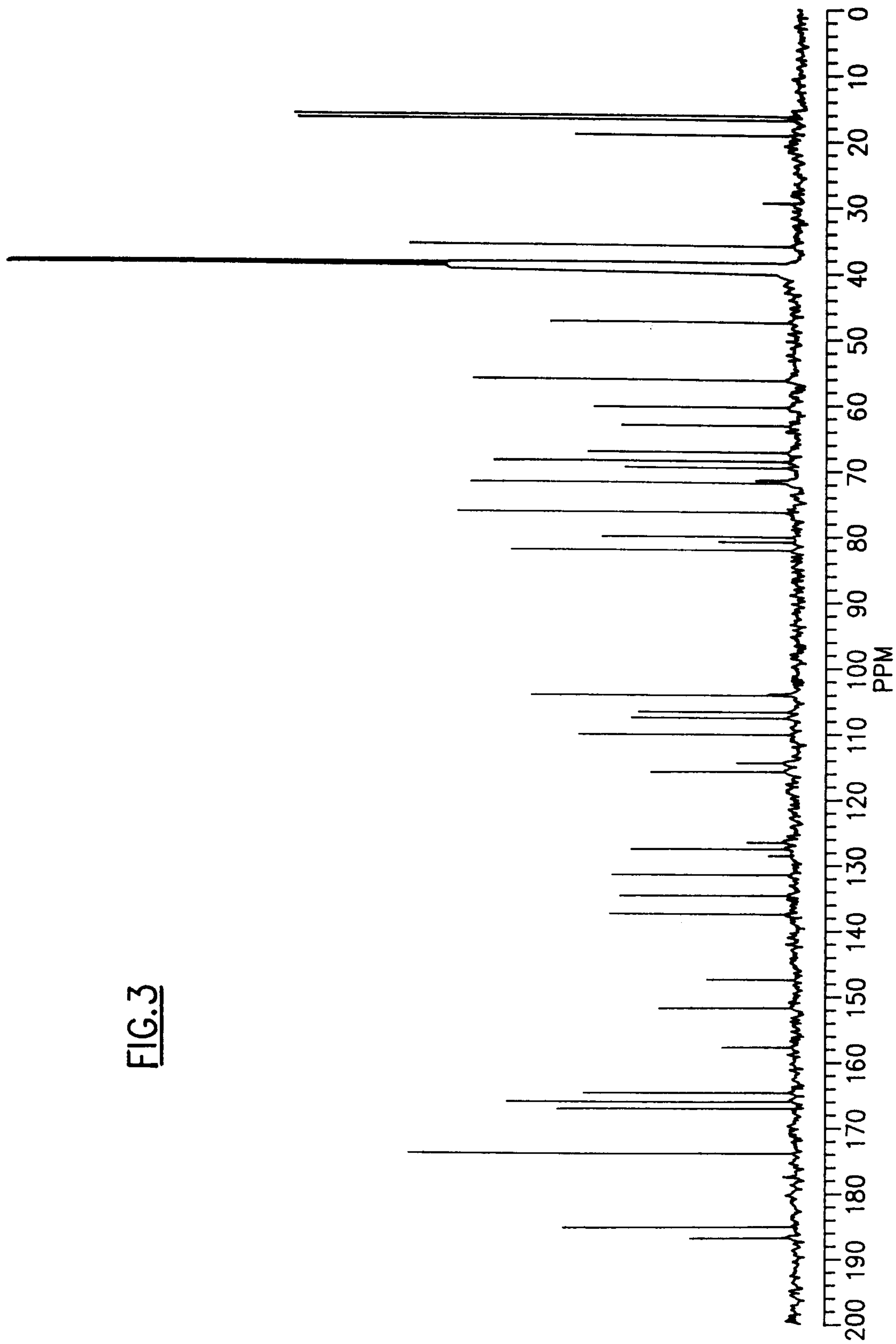

0
10
20
30
40
50
60
70
80
90
100
PPM
110
120
130
140
150
160
170
180
190
200

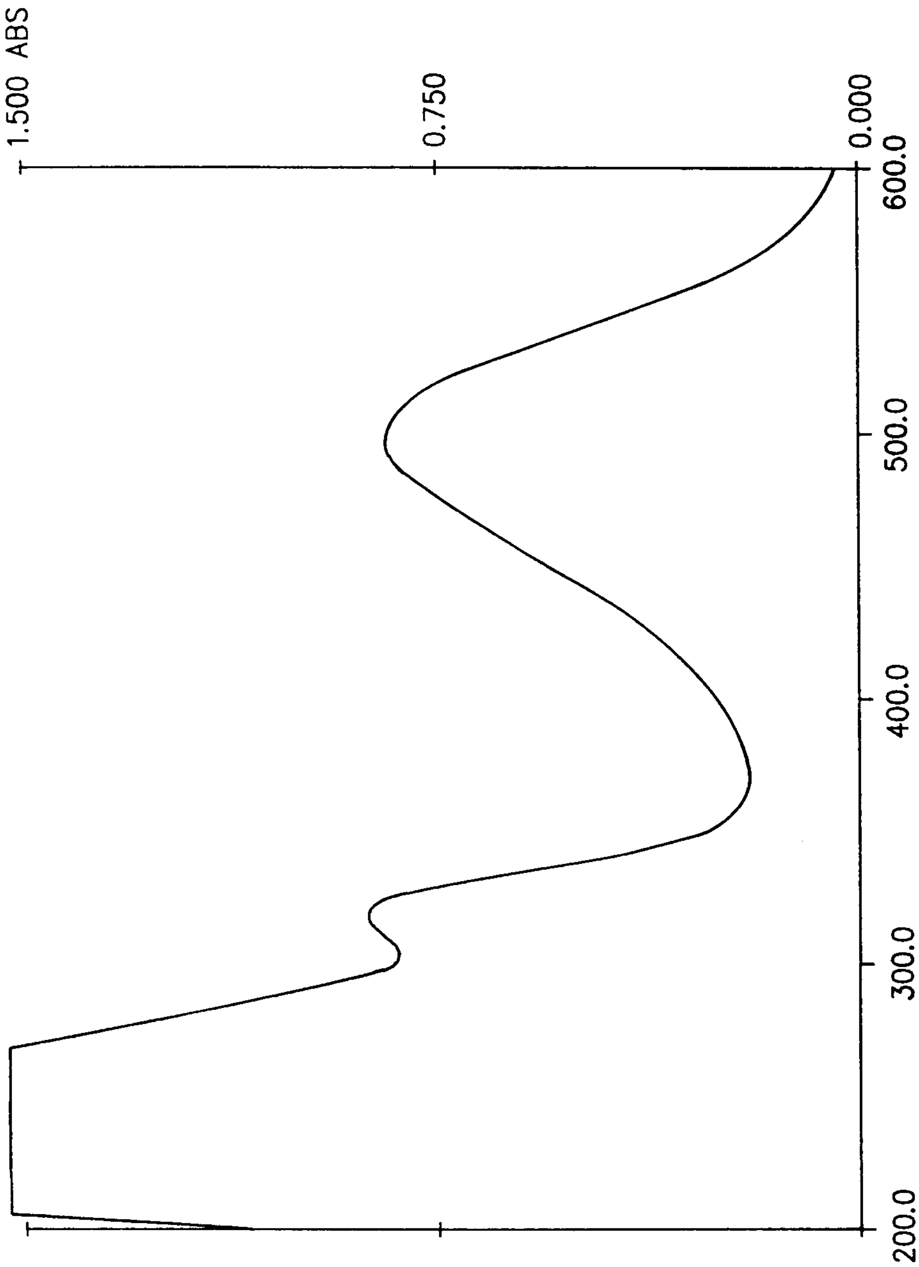


FIG.4

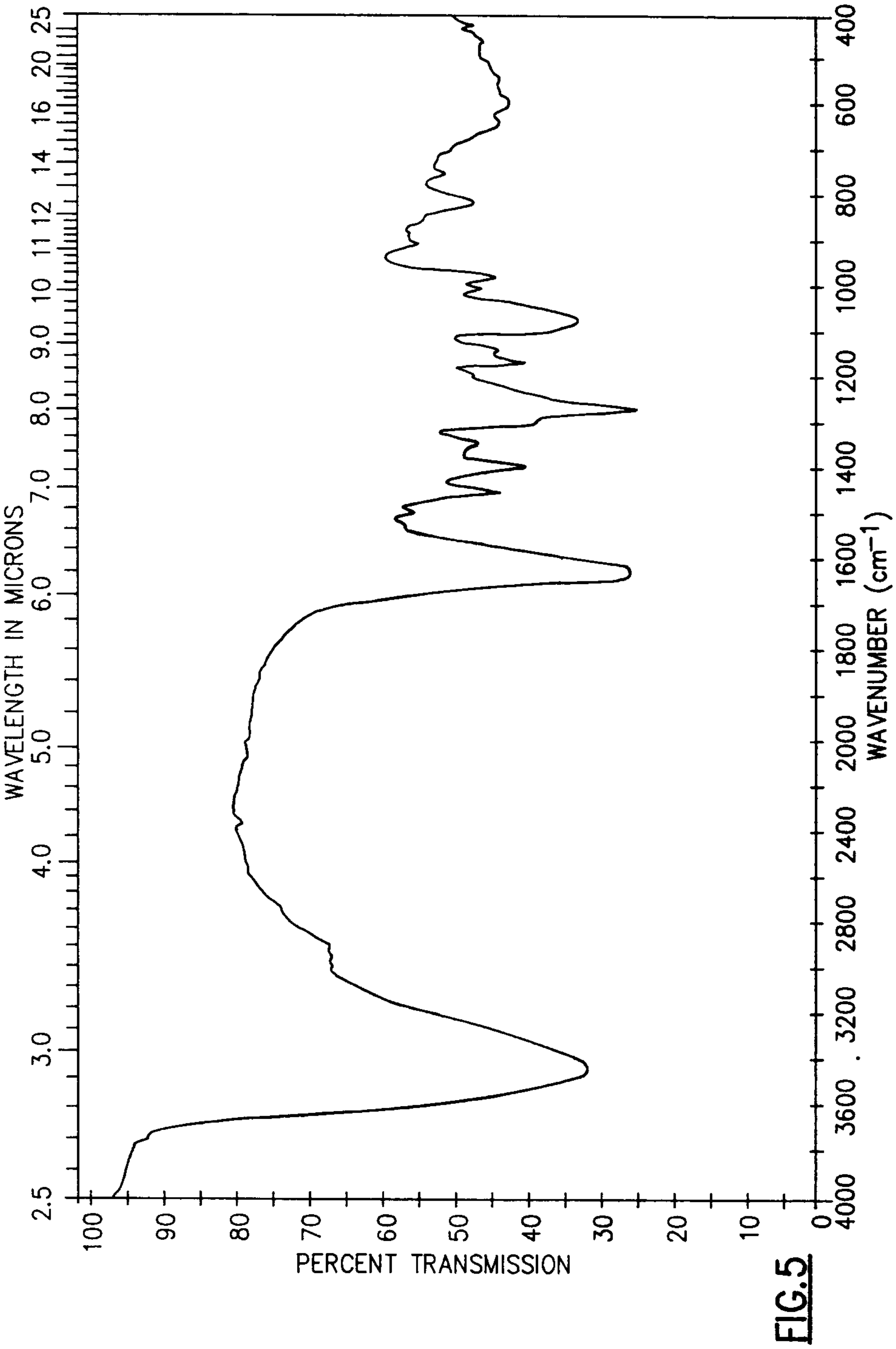
WAVELENGTH IN MICRONS
2.5 3.0 4.0 5.0 6.0 7.0 8.0 9.0 10 11 12 14 16 20 25
PERCENT TRANSMISSION
100 90 80 70 60 50 40 30 20 10 0
WAVENUMBER ($cm^{-1}$)
4000 3600 3200 2800 2400 2000 1800 1600 1400 1200 1000 800 600 400

FIG.5

FIG.6

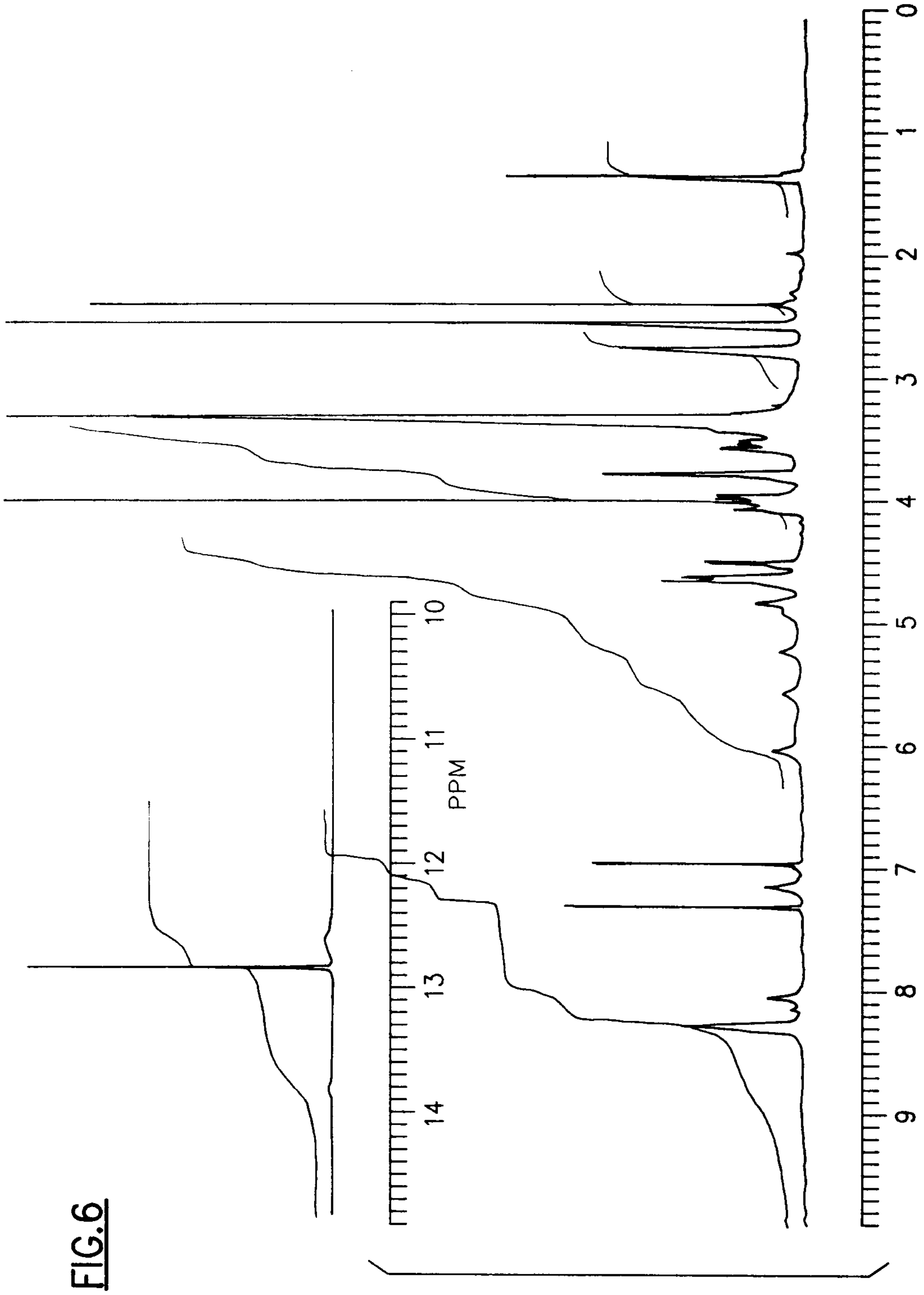

FIG.7

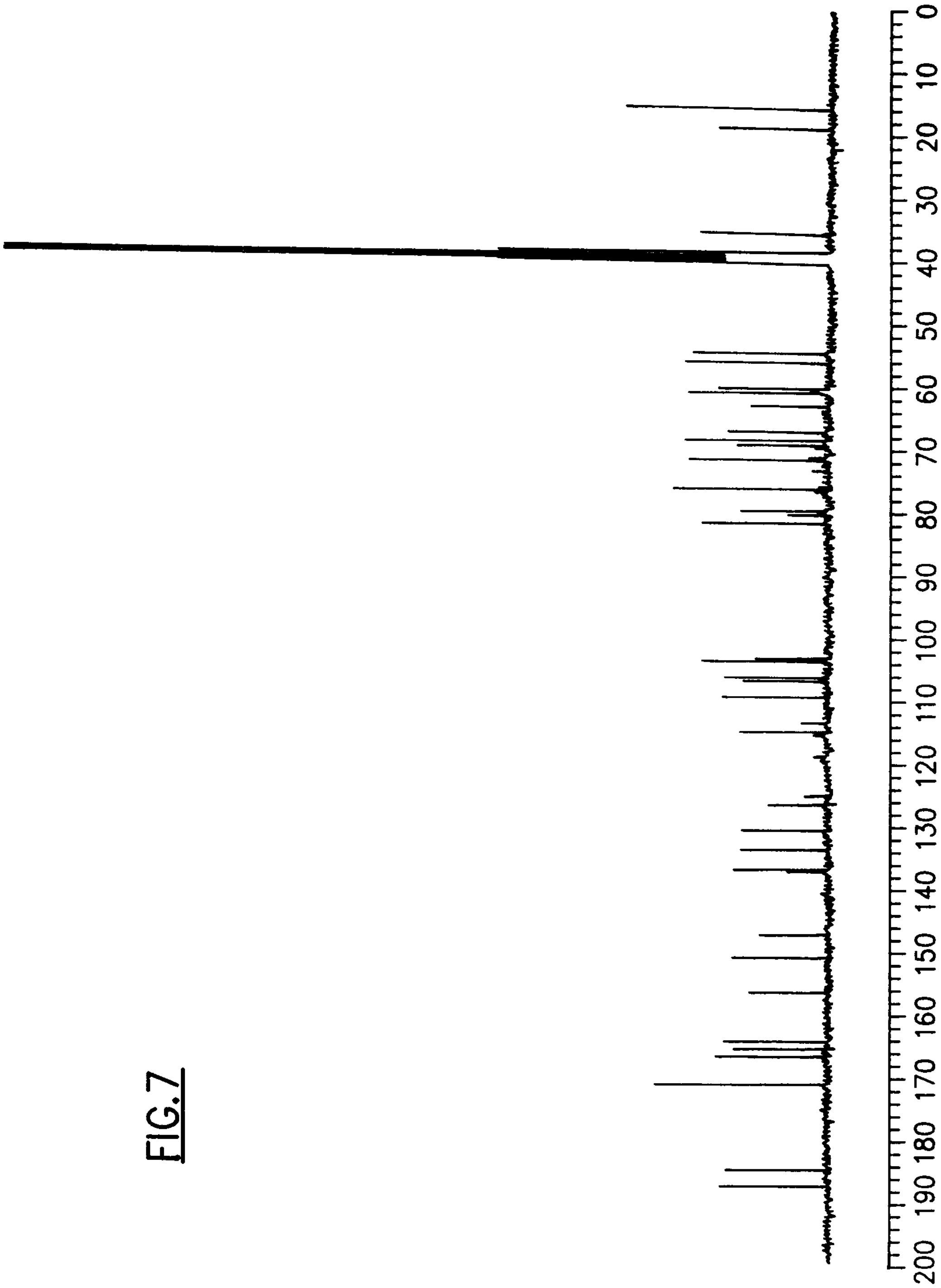
0
10
20
30
40
50
60
70
80
90
100
110
120
130
140
150
160
170
180
190
200
PPM

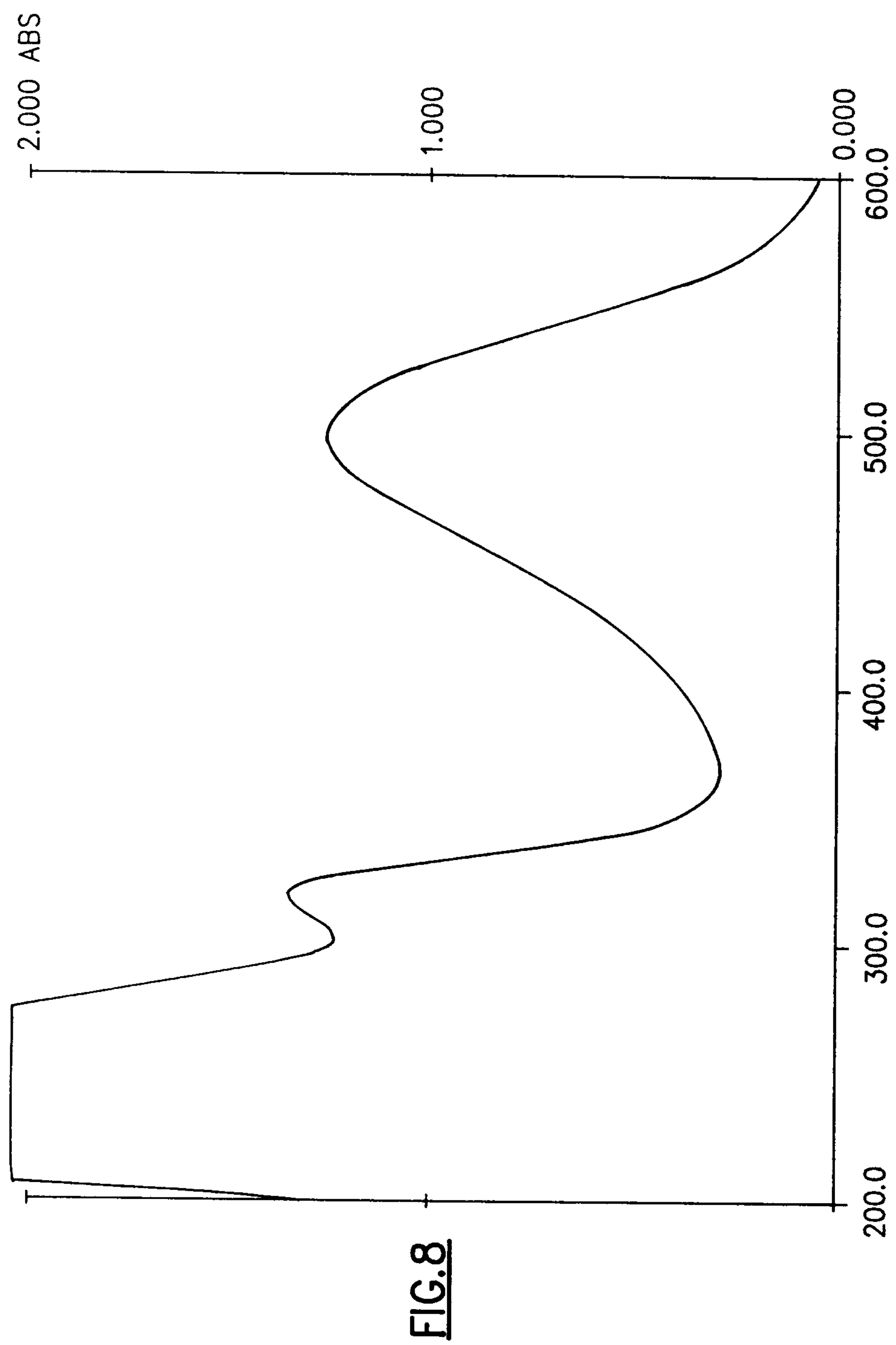


FIG.8